# EUROPEAN PATENT SPECIFICATION

(11) **EP 2 346 472 B1**
(45) Date of publication and mention of the grant of the patent: **22.11.2017**
(21) Application number: 09740939.5
(22) Date of filing: 08.10.2009
(51) Int. Cl.: A61K 6/027, A61K 6/097, A61K 8/00, A61Q 11/00

(54) **TOOTH REMINERALISATION**
ZAHNREMINERALISATION
REMINÉRALISATION DES DENTS

(30) Priority: 08.10.2008 US 247674
(43) Date of publication of application: 27.07.2011
(73) Proprietor: Biofilm Limited, Blantyre, Glasgow G72 0FB (GB); GlaxoSmithKline Consumer Healthcare (UK) IP Limited, Middlesex TW8 9GS (GB)
(72) Inventor: GIBSON, Alan Stewart, Johnstone PA5 8UB (GB); BURWELL, Anora Kirsten, Gainsville Florida 32609 (US)
(74) Representative: Morris, Miriam Elizabeth
(86) International application number: PCT/GB2009/051340
(87) International publication number: WO 2010/041073

(56) References cited:
- WO-A1-02/49578
- WO-A1-99/13852
- WO-A1-2006/055317
- US-A1- 2003 219 388
- DATABASE WPI Week 199823 Thomson Scientific, London, GB; AN 1998-261153 XP002573149 & WO 98/17236 A1 (SUNSTAR CHEM IND CO LTD) 30 April 1998 (1998-04-30) & JP 10 182393 A (SUNSTAR INC) 7 July 1998 (1998-07-07)

## Description

### FIELD OF THE INVENTION

The present invention relates to a film and a method of using a film for tooth remineralisation. More particularly, the present invention relates to a film comprising a bioactive glass and a water soluble polymer and a method of using such a film for tooth remineralisation.

### BACKGROUND OF THE INVENTION

It is well known that tooth structure comprises an inner dentine layer and an outer hard enamel layer that serves to protect the tooth. The enamel layer is composed of dense calcium hydroxyapatite mineral crystals; in the form of large "prismatic enamel" crystals surrounded by smaller crystals that fill in the "inter-prismatic" spaces. The formula of calcium hydroxy apatite is nominally Ca₅(PO₄)₃OH. The dentine however is composed of both hydroxy apatite, as well as structural proteins that result in a much softer and less dense structure than enamel and which is much easier to damage. The dentine structure also consists of pores or "tubules" which create openings from the tooth surface that run down into the pulp cavity of the tooth where the tooth nerve is located. Normally this softer porous dentine structure is covered by the denser enamel layer, or by the soft gingival tissue, which protects the nerve from outside stimuli. Tooth hypersensitivity is associated with the exposure of these tubules, through loss of the protective enamel layer or recession of the gums, that tends to increase with age. The recession of the gums can also be caused by overbrushing, as well as periodontal disease. The loss of enamel can occur as a result of acid erosion from acidic foods or cariogenic bacteria and abrasion from over brushing or other mechanical effects. Orthodontic brace wearers are also especially susceptible to loss of tooth enamel, leading to white spot lesions.

Demineralisation of tooth enamel tends to reveal tubules. Dentinal tubules are naturally present in the enamel and they provide for an osmotic flow of liquid between the inner pulp region of the tooth and the outer root surfaces. However, when demineralisation of the enamel layer occurs, these tubules tend to become exposed. When heat, cold, or pressure are applied, fluids tend to gain direct access to the tooth nerve causing pain. It is possible to alleviate this condition by coating the teeth with varnishes, lacquers etc., but this tends to be a short term remedy, since the lacquers become quickly worn away.

Bioactive glasses, alone or incorporated into oral care compositions, are known to remineralise teeth. When applied to the teeth they have the effect of rebuilding the hydroxy apatite structure. For example, US Patents 6,338,751 and 6,086,374, relate to oral care products which comprise remineralisation compositions (typically comprising calcium, phosphorous, silica and sodium ions in the form of a bioactive glass). The bioactive glass incorporated into these products releases remineralising ions onto the tooth's surface and helps occlude the open tubules, reducing tooth sensitivity, and rebuilding the enamel layer. However, a disadvantage of these compositions is that the contact time for toothpastes or dentifrices is limited to the time of brushing and so may only be a minute or two.

US 6,365,132 and WO 99/13852, detail methods of whitening teeth by contacting the tooth surface with an effective amount of particulate bioactive glass. However, the compositions defined are obtrusive (i.e. noticeable) and not suitable for use in all situations.

US 6,190,643, incorporated herein by reference, discloses a method for reducing the viability of detrimental oral microorganisms and for the whitening of teeth by treatment with bioactive glass.

Patent publication WO 02/49578 Henkel Corporation (corresponding to US2003/0219388), discloses a water-soluble or water-swellable film containing various soluble calcium salts for remineralisation of teeth. However, these salts are "poorly soluble" in water and the hydroxy apatite is preformed in the film resulting in the limited ability to remineralise the tooth surface.

Moreover, many prior art documents point away from the use of bioactive glasses in aqueous based systems due to their inherent bioactivity. For example, we refer to Chapter 3 of "An Introduction to Bioceramics", L.L Hench and J.Wilson, incorporated herein by reference, which states that the alkaline species in the glass can be depleted within a few minutes of exposure to an aqueous solution.

Additionally, WO 2006/055317, describes dental compositions that release calcium and phosphorus into the oral cavity over a sustained period. These products have been engineered to remain on the tooth surface for a number of months and can be prepared from polymerisable materials which harden on the tooth surface. Other compositions are prepared from solvent based (ethanol) formulations.

It is an object of at least one aspect of the present invention to obviate or mitigate at least one or more of the aforementioned problems.

It is a further object of at least one aspect of the present invention to provide a film capable of tooth remineralisation.

It is a further object of at least one aspect of the present invention to provide a method of tooth remineralisation using a film.

### SUMMARY OF THE INVENTION

According to a first aspect of the present invention there is provided a film for use in an oral cavity, said film comprising:
a water soluble polymeric film former; and
a bioactive glass.

According to a second aspect of the present invention there is provided a film for use in an oral cavity, said film comprising:
a water soluble polymeric film former; and
a bioactive glass;
wherein the film is capable of adhering to at least one tooth in the oral cavity for a maximum time of about 60 minutes before the film dissolves or substantially dissolves and wherein said film is capable of performing tooth remineralisation.

According to a third aspect of the present invention there is provided a film for use in an oral cavity, said film comprising:
a water soluble polymeric film former; and
a bioactive glass;
wherein the film is capable of dissolving or at least substantially dissolving within a maximum time of about 60 minutes and wherein said film is capable of performing tooth remineralisation.

Typically, the film may be capable of performing tooth remineralisation on application to a tooth or a number of teeth. Generally speaking, the present invention may reside in the provision of a remineralisation composition comprising a bioactive glass in the form of a thin film, which may be adhered to one or more teeth, and provides a convenient and unobtrusive method of generating a high and sustained local concentration of remineralising components on a tooth surface.

When hydrated, the film of the present invention may release remineralising ions forming, for example, a stable crystalline hydroxy carbonate apatite layer on a tooth surface, providing long term reduction of tooth sensitivity.

The film may also comprise a naturally derived film former which may, for example, be chemically modified. For example, the naturally derived chemically modified film former may be any suitable water soluble polymer but preferably a naturally derived non ionic cellulose derivative such as hydroxypropyl cellulose which allows a pH range of between about 6.0 and 11.0, between about 7.0 and 10.5 or preferably between about 7.5 and 9.5, which is optimal for hydroxy carbonate apatite mineral formation.

It has also advantageously been found that the film of the present invention preferentially sticks to the teeth without sticking to the soft tissue inside the mouth. Although not wishing to be bound to theory the water soluble polymeric film former such as hydroxypropyl cellulose may be classified as a surface active polymer. Water based solutions of the water soluble polymeric film former may display greatly reduced surface and interfacial tension. Hydrated layers of the water soluble polymeric film former may effectively lubricate the soft tissue inside a mouth, thereby allowing the hydrated water soluble polymeric film former to easily slip over, for example, the inside of the lip.

The film of the present invention may also be extremely useful as the film is capable of accumulating and hydrating around orthodontic brackets delivering a high concentration of the ions required to help generate hydroxy carbonate apatite at a point of need.

By water soluble herein in reference to the water soluble polymeric film former means any polymeric material which forms a solution or colloidal solution, where the approximate volume of solvent in millilitres per gram of solute is in the range of about 0.01 to 10,000.

The bioactive glass may, for example, be a particulate glass which may be capable of releasing a remineralisation composition. The bioactive glass may be an inorganic glass having an oxide of silicon as its major component and which may be capable of bonding with growing tissue when in contact with physiological fluids. The bioactive glass contains the essential ions of, for example, calcium and phosphorus and as the saliva dissolves the film (e.g. a strip) these ions are released to help form hydroxy carbonate apatite on the tooth. The film may therefore comprise: calcium; phosphorous; silica and/or sodium atoms and/or ions, or any combination thereof, in the form of a bioactive glass.

The film may form a layer of hydroxy carbonate apatite *in vitro* when placed in a body fluid or a simulated body fluid. Conveniently, the film does not trigger any significant adverse immune response in the body, such as in the oral cavity.

The film comprises a water soluble polymeric film former in an amount of about 1 - 90% by weight, about 20 - 80% by weight, about 30 - 70% by weight, about 40 - 80% by weight or about 50 - 60% by weight. Alternatively, the film may comprise a water soluble polymeric film former in an amount of at least about 10% by weight, at least about 30% by weight, at least about 50% by weight, at least about 70% by weight or at least about 90% by weight.

The film comprises bioactive glass in an amount of about 10 - 90% by weight, about 20 - 80% by weight, about 30 - 70% by weight, about 40 - 80% by weight or about 50 - 60% by weight. Alternatively, the film may comprise bioactive glass in an amount of at least about 10% by weight, about 30% by weight, about 50% by weight, about 70% by weight or about 90% by weight. Generally speaking, the particulate glass may comprise from about 1 - 60%, about 1 - 30%, typically about 2 - 25%, especially about 5 - 20%, and notably about 8 - 16% by weight of the film.

A typical composition for the film is 40 - 80% by weight silicon dioxide (SiO₂), about 0 - 35% by weight of sodium oxide (Na₂O), about 4 - 46% by weight calcium oxide (CaO) and about 1-15% by weight phosphorus oxide (P₂O₅). When made by a traditional glass melting process, preferred ranges may be:
about 40 - 60% by wt. silica;
about 10 - 30% by wt. sodium oxide;
about 10 - 30% by wt. calcium oxide; and
about 2 - 8% by wt. phosphorus oxide.

In particular embodiments, the water soluble polymeric film former may be made from a polymer having an average molecular weight range of about 1,000 - 1,500,000, about 1,000 - 500,000, about 50,000 - 500,000, about 100,000 - 200,000, about 1,000 - 100,000, about 1,000 - 50,000, about 1,000 - 20,000 or about 10,000 - 20,000. Preferably, the average molecular weight may be about 50,000 - 250,000 or most preferably about 140,000. The average molecular weight may be determined by size exclusion chromatography.

As well as providing a matrix for the bioactive glass, the silica may act as an abrasive when a gelled film is removed from the teeth by brushing.

The bioactive glass may be made by a traditional glass melting process (when sodium oxide is usually present); or by a precipitation or gelation process referred to as a "sol-gel" process in prior art (when sodium oxide may be absent). Other components such as calcium fluoride, boron oxide, aluminium oxide, magnesium oxide and potassium oxide may also be included.

The bioactive glass may be a calcium and phosphorus releasing glass including, for example, calcium and phosphorus in a glass that preferably allows them to be released when placed in the oral environment. Such glasses have been described in the literature as "remineralising" or, with respect to medical applications, "bioactive." Such glasses may be melt or sol-gel derived. Such glasses may also be amorphous or include one or more crystalline phases (i.e., partially crystalline, sometimes described as "glass-ceramics").

Remineralising or bioactive glasses are well known to one of skill in the art, and typical glasses are described, for example, in U.S. Pat. Nos. 4,698,318 (Vogel et al.), 5,074,916 (Hench et al.), 5,162,267 (Smyth), 5,296,026 (Monroe et al.), 6,338,751 (Litkowski et al.), and 6,709,744 (Day et al.), and U.S. Patent Application Publication Nos. 2003/0167967 (Narhi et al.), 2004/0241238 (Sepulveda et al.), and 2004/0065228 (Kessler et al.). Exemplary remineralising or bioactive glasses are available, for example, from NovaMin (US) under their trade names and also under the trade designations CERABONE A/W from Nippon Electric Glass Co., Ltd. (Shiga, Japan), BIOVERIT as described by Vogel in Introduction to Bioceramics, L.L. Hench and J. Wilson, eds., World Scientific Publishing (1993), 45S5 and 45S5F as described by Hench and Andersson in Introduction to Bioceramics, L.L. Hench and J. Wilson, eds., World Scientific Publishing (1993). The bioactive releasing glass may not include high levels of aluminum oxide (e.g. alumina), which is known to hinder bone mending in medical applications. Such glasses without high levels of aluminum oxide may include less than about 5%, and sometimes less than about 3%, 2%, or even about 1% by weight aluminum oxide. In contrast, ionomer glass compositions generally rely on a sufficiently high level of leachable aluminum ions for the ionomeric crosslinking reaction, typically 10 - 45% by weight Al₂O₃.

In some embodiments, the bioactive glass may include about 35% to 60% by weight silica, and preferably about 40% to 60% by weight silica. In some embodiments, the bioactive glass includes less than about 20%, and sometimes less than about 15%, 10%, 5%, 3%, or even 1 % by weight silica.

In some embodiments, the bioactive glass may include at least about 15%, and sometimes at least about 20%, 25%, 30%, 35%, or even 40% by weight phosphorus pentoxide (P₂O₅). In such embodiments, bioactive releasing glass may include at most about 80%, and sometimes at most about 75%, 70%, 65%, 60%, 55%, 50%, 45%, or even about 40% by weight phosphorus pentoxide (P₂O₅).

The bioactive glass may include less than about 20%, and sometimes less than about 15%, 12%, 8%, or even about 6% by weight phosphorus pentoxide (P₂O₅). In such embodiments, the bioactive glass includes at least about 1%, and sometimes at least about 2%, or even about 3% by weight phosphorus pentoxide (P₂O₅).

In some embodiments, the bioactive glass may include at least about 10%, and sometimes at least about 15%, 20%, 25%, or even about 30% by weight calcium oxide. In such embodiments, the bioactive glass may include at most about 70%, and sometimes at most about 60%, 50%, 40%, or even about 35% by weight calcium oxide.

The bioactive glass optionally includes at most about 25%, and sometimes at most about 20%, 15%, 10%, or even about 5% by weight fluoride.

In some embodiments, the bioactive glass optionally includes at most about 60%, and sometimes at most about 55%, 50%, 45%, 40%, 35%, or even about 30% by weight of SrO, MgO, BaO, ZnO, or combinations thereof. In some embodiments, the bioactive glass optionally includes at least about 0.5%, and sometimes at least about 1%, 5%, 10%, 15%, or even 20% by weight of SrO, MgO, BaO, ZnO, or combinations thereof. The bioactive glass optionally includes at most about 40%, and sometimes at most about 35%, 30%, 25%, 20%, 15%, 10%, or even about 5% by weight rare earth oxide. The bioactive glass optionally includes at most about 45%, and sometimes at most about 40%, 30%, 20%, 10%, 8%, 6%, 4%, 3%, or even about 2% by weight of Li₂O, Na₂O, K₂O, or combinations thereof.

The bioactive glass optionally includes at most about 40%, and sometimes at most about 30%, 25%, 20%, 15%, 10%, or even about 5% by weight of B₂O₃.

The bioactive glass may include less than about 15%, and sometimes less than about 10%, 5%, or even about 2% by weight of ZrO₂.

The bioactive glass may include about 40 - 60% by weight SiO₂, about 10-35% by weight CaO, about 1 - 20% by weight P₂O₅ about 0 - 35% by weight Na₂O, and less than about 5% by weight Al₂O₃.

The bioactive glass may include about 10 - 70% by weight CaO; about 20 - 60% by weight P₂O₅; less than about 3% by weight Al₂O₃; about 0 - 50% by weight of SrO, MgO, BaO, ZnO, or combinations thereof; and less than about 10% by weight Li₂O, Na₂O, and K₂O combined.

The bioactive glass may include about 10- 70% by weight CaO; about 20 - 50% by weight P₂O₅; less than about 3% by weight Al₂O₃; about 0 - 50% by weight of SrO, MgO, BaO, ZnO, or combinations thereof; and less than about 10% by weight Li₂O, Na₂O, and K₂O combined.

The bioactive glass may include about 10 - 50% by weight CaO, at least 15% and less than about 50% by weight P₂O₅, less than about 3% by weight Al₂O₃, less than about 10% by weight Li₂O, Na₂O, and K₂O combined, and about 0-60% by weight of SrO, MgO, BaO, ZnO, or combinations thereof.

The glass may be in a variety of finely divided forms including particles, fibers, or platelets. The preferred average particle size for dental and orthodontic applications is less than about 50 micrometers, more preferably less than about 10 micrometers, most preferably less than about 3 micrometers. Nanoscale sizes (e.g. less than about 0.5 micrometers) are also highly preferred. Combinations of different size ranges can also be used.

The bioactive glass can optionally be surface treated (e.g. with silane; acid- or acid-methacrylate monomers, oligomers, or polymers; other polymers, etc.) as described herein below. Such surface treatments can result, for example, in improved bonding of the particles to a matrix. Preferably, the glass is surface treated by methods similar to those described, for example, in U.S. Pat. No. 5,332,429 (Mitra). In brief, the glass can be surface treated by combining the glass with one or more liquids having dissolved, dispersed, or suspended therein, a surface treating agent (e.g. fluoride ion precursors, silanes, titanates, etc). Optionally the one or more liquids include water, and if an aqueous liquid is used, it can be acidic or basic. Once treated, at least a portion of the one or more liquids can be removed from the surface treated glass using any convenient technique (e.g. spray drying, oven drying, gap drying, lyophilising, and combinations thereof). See, for example, U.S. Pat. No. 5,980,697 (Kolb et al.) for a description of gap drying. In one embodiment, the treated glass can be oven dried, typically at drying temperatures of about 30 °C to about 100 °C, for example, overnight. The surface treated glass can be further heated as desired. The treated and dried glass can then be screened or lightly comminuted to break up agglomerates.

Typically, average particle size of the bioactive glass may have an effect on the release rate of ions when in contact with water and may typically be less than about 100 microns, about 75 microns, about 50 microns, about 20 microns, usually less than about 10 microns, and often less than about 5 microns. Alternatively, the average particle size of the bioactive glass may range from about 0.01 - 200 microns; about 0.01 - 50 microns; about 0.01 - 20 microns; about 0.1 - 10 microns; or about 0.1 - 5 microns.

In particular embodiments, the 50^{th} percentile diameter of the bioactive glass may usually be in the range of about 2 - 20 microns; or for example, about 5 - 10 microns. Suitable bioactive glasses are available under the NovaMin® trademark but other bioactive glasses may be used.

The bioactive glass may be mixed with other materials such as any one or combination of the following: sweeteners; flavourings; antimicrobial agents; plasticising agents; colouring agents; saliva stimulating agents; cooling agents; surfactants; stabilising agents; emulsifying agents; thickening agents; preservatives; bulking agents; humectants; vitamins; minerals; fluorides; and fillers.

The film may be in the form of a thin strip suitable for oral consumption.

The film, preferably in the form of a strip, is intended to swell or dissolve in an oral cavity releasing the remineralisation composition over a period of time. The film is intended to be adhered to a tooth surface, either by its own properties or using an adhesive. The particulate glass in the strip may be provided in a single layer, incorporated into one or more layers of a multilayer product, or alternatively, may simply be deposited onto the surface of a strip of film former (preferably of water-dispersible edible material).

Typically, the film may be a strip wherein the strip may be substantially rectangular in shape, but any shape of strip (e.g. substantially rounded rectangle, substantially oval, substantially square, substantially circular) may be used. The strip may have a minimum thickness of about 10 µm to allow ease of handling. The strip may have a preferred maximum thickness of about 500 µm, or more preferably about 200 µm, so that the physical dimensions of the strip do not prevent the body from dissolving in a desired period of time in an oral cavity e.g. by the action of saliva. Generally, the thickness of the strip may be in the range of about 20 to 100 µm, and more usually about 40 to 80 µm.

The width of the strip may be approximately about 0.05 - 5 cm or preferably about 1 - 2.5 cm, and the length may be about 0.05 - 10 cm or preferably about 4 - 8 cm.

Generally, the strip will be intended for application to one or two or more teeth but smaller pieces may be incorporated into other products (e.g. dentifrices, toothpastes etc.).

The strip may be dry, by which is meant that the strip is dry to the touch. However, the strip may contain approximately about 1 to 20% by weight, or about 1 to 15% by weight, of residual water, which remains after the manufacturing process. When dry, the film effectively adheres to the tooth (by absorbing water from the tooth surface and oral cavity) and is thus anchored in place for a period of time. This helps provide a long contact time (compared, for example, to toothpastes).

The strip may rapidly dissolve in water/saliva in the oral cavity. By rapid dissolution it is meant that the water-soluble components (e.g. the water-soluble polymeric film former) of the strip preferably dissolves, or substantially dissolves, in a time of about 1 to 30 minutes, about 5 to 20 minutes, about 5 to 15 minutes or about 5 to 10 minutes. Typically, the time for the dissolution of a strip in an oral cavity may be about 60 minutes maximum and the minimum time would be about 1 second. Generally, the strip substantially dissolves in about 10 seconds to about 10 minutes. Within that time, release of a substantial portion of the remineralisation composition comprising the bioactive glass will have occurred.

The film is formed of edible material. By "edible material" is meant a material that is acceptable for consumption by a user. Normally, this is a material approved for use by the appropriate regulatory authorities.

The strip is formed from any suitable edible water-soluble polymeric materials, such as any one of or combination of the following: methyl celluloses, (e.g. sodium carboxymethyl cellulose), hydroxypropylmethyl cellulose, hydroxyethyl cellulose, hydroxypropyl cellulose, carboxymethyl cellulose. Any suitable combination of the above compounds such as a mixture of two, three, four, or more, of the materials may be employed. Additionally, different layers in a multi-layer strip may comprise different combinations of materials.

It is preferable to use a non-ionic film former, such as a cellulose derivative (for example hydroxypropyl cellulose, hydroxypropyl methylcellulose and the like). The non-ionic nature of the film former avoids undesired reaction with the remineralisation composition.

In use, the film hydrates and absorbs water and swells, thereby allowing release of the remineralisation ions from the film onto the tooth's surface. The film former may simply swell but remain essentially intact for later removal or the film former may become dissolved or dispersed in the saliva within the oral cavity.

The remineralisation composition itself has been found to exhibit useful antimicrobial properties. However, the strip may also comprise antimicrobial agents (i.e. bactericides) in any suitable form. The antimicrobial agents may be present in an amount up to the maximum levels permitted by appropriate regulatory authorities. For example, any one or combination of the following antimicrobial agents may be used: triclosan; cetyl pyridinium chloride (CPC); domiphen bromide; quaternary ammonium salts; sanguinarine; suitable fluorides; alexidine; octonidine; and EDTA.

Flavourings may also be included. Suitable flavourings may exhibit fragrant properties. Suitable flavourings may be added during the manufacturing process in the form of flavoured oils. The flavourings may form amounts of about 0 - 30 wt.% or about 0 - 10 wt.% of film.

The flavourings may be chosen from natural oils, synthetic flavour oils, flavouring aromatics, oleo resins and extracts derived from plants, leaves, flowers, fruits and so forth, and combinations thereof.

Flavour oils which may be used include any one or combination of the following: spearmint oil; cinnamon oil; peppermint oil; clove oil; bay oil; thyme oil; cedar leaf oil; oil of nutmeg; oil of sage; and oil of bitter almonds.

Also useful are artificial, natural or synthetic fruit flavours such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavourings can be used individually or in a mixture. Commonly used flavours include mints, such as peppermint, artificial vanilla, cinnamon derivatives, and various fruit flavours, whether employed individually or in admixture. Flavourings such as aldehydes and esters including cinnamyl acetate, cinnamaldehyde, citral, diethylacetal, dihydrocarvyl acetate, eugenyl formate, and p-methylanisole, and so forth may also be used.

Further examples of aldehyde flavourings include, but are not limited to any one or combination of the following: acetaldehyde (apple); benzaldehyde (cherry, almond); cinnamic aldehyde (cinnamon); citral, i.e. alpha citral (lemon, lime); neral, i.e. beta citral (lemon, lime); decanal (orange, lemon); ethyl vanillin (vanilla, cream); helliotropine, i.e. piperonal (vanilla, cream); vanillin (vanilla, cream); alpha-amyl cinnamaldehyde (spicy fruity flavours); butyraldehyde (butter, cheese); valeraldehyde (butter, cheese); citronellal; decanal (citrus fruits); aldehyde C-8 (citrus fruits); aldehyde C-9 (citrus fruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 2,6-dimethyloctanal (green fruit); and 2-dodecenal (citrus, mandarin); cherry; grape; mixtures thereof; and the like.

As required, the film may comprise a sweetening agent or a combination of sweetening agents. The sweetening agents may be present in an amount of less than about 30 wt.%, but more typically less than about 20 wt.%.

Typical sweeteners include monosaccharides, disaccharides and polysaccharides such as any one or combination of the following: xylose; ribose; glucose (dextrose); mannose; galactose; fructose (i.e. levulose); sucrose (i.e. sugar); and maltose; an invert sugar (i.e. a mixture of fructose and glucose derived from sucrose); partially hydrolysed starch; corn syrup solids; dihydrochalcones; monellin; steviosides; and glycyrrhizin.

Additionally, water-soluble artificial sweeteners such as soluble saccharin salts may be used. For example, any one or combination of the following artificial sweeteners may be used: sodium or calcium saccharin salts; cyclamate salts; sodium, ammonium or calcium salts of 3,4-dihydro-6-methyl-1,2,3-oxathiazine-4-one-2,2-dioxide; potassium salts of 3,4-dihydro-6-methyl-1,2,3-oxathiazine-4-one-2,2-dioxide (acesulfame-K); sucralose; and the free acid form of saccharin.

Moreover, dipeptide based sweeteners such as any one or combination of the following may be used: L-aspartic acid derived sweeteners, such as L-aspartyl-L-phenylalanine methyl ester (aspartame); L-alpha-aspartyl-N-(2,2,4,4-tetramethyl-3-thietanyl)-D-alaninamide hydrate; methyl esters of L-aspartyl-L-phenylglycerin and L-aspartyl-L-2,5-dihydrophenyl-glycine; L-aspartyl-2,5-dihydro-L-phenylalanine; and L-aspartyl-L-(1-cyclohexyen)-alanine.

Water-soluble sweeteners derived from naturally occurring water-soluble sweeteners, such as a chlorinated derivative of ordinary sugar (i.e. sucrose) may also be used.

Furthermore, protein based sweeteners such as thaumatoccous danielli (i.e. Thaumatin I and II) may be used.

Plasticising agents may also be included. The plasticising agents may be present in an amount of about 0 - 25 wt.% or about 0 to 15 wt.% of the film. For example, plasticising agents such as triacetin, monoacetin, diacetin and polyols e.g. glycerol, sorbitol and mannitol may be used.

Typically, the manufacturing process to form the film may involve dispersing the bioactive glass in a polymer solution containing all other ingredients. Alternatively, the solvent could be omitted if a suitable hot melt process is used.

It is surprising that the film of the present invention can be produced from an aqueous mixture of its components. Previous expectation was that in the aqueous mixture, the particulate glass would prematurely release ions which would form hydroxyapatite in the film. However, it has been surprisingly found that the aqueous production environment does not significantly detract from the bioactivity of the bioactive glass; providing that extended processing times are avoided.

The ratio of solvent to the particulate glass and other ingredients may be altered during processing to obtain the proper viscosity for film formation. The resulting liquid may be made homogenous by appropriate mixing and step-wise addition of water-soluble polymeric film former material. A film may then be formed using proprietary procedures wherein the obtained liquid containing the bioactive glass may be deposited (e.g. by extrusion) or other film forming techniques known to those skilled in the art onto carrier paper or a continuous belt, which may be used as a support as the wet film is carried through a drier. The film support material may be a polymeric film or paper but is preferably a low density polyethylene coated craft paper.

To avoid adverse reactions, it is preferred to minimise the time between formation of the aqueous solution/suspension and formation of the film.

The drier may comprise a tunnel of warm low humidity air or utilize other heat sources, such as Infra Red (IR) lamps, to evaporate the solvent and form a dry web. Alternatively, the film may be cast into sheets and dried in an oven. Typically, the maximum air temperature in a drier or oven may be about 100 °C and the minimum temperature may be about 20 °C. Preferably, the maximum air temperature in a drier or oven would be about 80 °C and a minimum temperature may be 30 °C.

The resulting film may then be cut and packed directly, or stored in reel or sheet format for future processing.

The mixture containing the bioactive glass, solvent and the water-soluble edible polymeric material may be dried to form a laminar body and thereafter formed into a strip using standard techniques.

In terms of delivery of remineralisation composition, a strip will typically contain about 1 to 100 mg, about 1 to 50 mg, about 1 to 20 mg or about 1 to 10 mg of bioactive glass per tooth or, for example, or about 1 to 100 mg or about 3 to 30 mg within a film for use on multiple teeth. A particular benefit of the film is that it allows teeth to be completely covered with a uniform or substantially uniform layer of remineralisation material.

The film may also contain other remineralising agents, such as fluoride or other agents that release calcium and phosphorous that can be available for tooth remineralisation, for example, during an acid attack of the tooth surface. Examples of other remineralising agents can include, but are not limited to any one or combination of the following: sodium fluoride (NaF); sodium monofluorophosphate (MFP); stannous fluoride; amorphous calcium phosphate (ACP); casein phosphopeptide (Recaldent®) or other soluble calcium phosphate compositions.

The film may also be used for the prevention and treatment of gingivitis (gum disease) and root caries and has been demonstrated to inhibit the growth of the oral bacteria associated with caries development and periodontal disease. A particular source of tooth demineralisation is associated with the wearing of orthodontic braces, where contact between the tooth and the (usually metal) brace leads to erosion of tooth enamel by creating interfaces where cariogenic bacteria can live. The demineralised areas are referred to as "white spot lesions" because the demineralised enamel is much whiter than the surrounding enamel. The film of the present invention may thus be used in a prophylactic or therapeutic capacity.

According to a fourth aspect of the present invention there is provided a dental composition for use in the oral cavity, said dental composition comprising:
a water soluble polymeric film former; and
a bioactive glass.

The water soluble polymeric film former and bioactive glass may be as defined above.

Typically, the dental composition may be in the form of a film (e.g. a strip). According to a fifth aspect of the present invention there is provided a film for use in the oral cavity for tooth remineralisation, which comprises:
a water soluble polymeric film former; and
a particulate glass comprising calcium oxide, silicon oxide and/or phosphorous oxide, which releases calcium and phosphorous ionic moieties in contact with water and is effective to produce crystalline hydroxyapatite on a tooth.

The particulate glass is a bioactive glass.

The water soluble polymeric film former and bioactive glass may be as defined above.

According to a seventh aspect of the present invention there is provided a method of forming a film for use in the oral cavity and capable of performing tooth remineralisation, said method comprising:
providing a water soluble polymeric film former; and
providing a bioactive glass;
mixing the water soluble polymeric film former and the bioactive glass; and
wherein the mixture of the water soluble polymeric film former and the bioactive glass is capable of being formed into a film capable of performing tooth remineralisation.

Typically, the film may be as defined in the first aspect of the present invention. The water soluble polymeric film former and bioactive glass may therefore be as defined above.

The method may comprise adding the bioactive glass to an aqueous mix containing a water soluble polymer. This mix may also contain plasticisers, humectants, flavours, emulsifiers, fillers, sweeteners and colours or combinations thereof.

A resulting liquid mixture may then be passed through a high shear mixer to produce a homogenous dispersion which in turn may be de-aerated before being coated onto a carrier paper and air dried at above room temperatures not exceeding, for example, about 60 °C.

Although not wishing to be bound by theory, the point of addition of the bio-active glass to the polymer solution is thought to be important since adding it after the polymer limits the amount of free water in the system. The bioactive glass may be held in this environment for about 1 - 120 hours or more preferably about 1 - 60 hours before the water content is reduced to about 5 - 70%, 5 - 50%, about 5 - 25% or about 20% or about 10% by weight. At this point the water activity of the dried film is typically below about, for example, 0.8, and usually below about 0.6.

The unexpected and surprising result is that the bioactive glass remains relatively intact during the manufacturing process, maintaining a high level of reactivity through to the finished product. The preservation of reactivity is demonstrated by a rise in pH when the films are dissolved in water, caused by ions migrating from the glass, and is backed up by FTIR studies which indicate that the glass structure remains substantially intact through the manufacturing process (this is demonstrated by the presence of bands associated with Si-O bonds and the relative absence of those associated with the hydroxcarbonate apatite structure).

A number of advantages may be gained by preparing the films from water soluble polymers and an aqueous casting medium as defined in the present invention. For example,
- Water soluble polymers dissolve/disperse rapidly in the mouth providing a high concentration of the bioactive glass, and thus the ions required to form hydroxycarbonate apatite, on the tooth surface.
- Using an aqueous base (i.e. water soluble polymer) removes the need for costly non-aqueous solvents and the additional capital costs associated with solvent recovery or ensuring equipment is explosion proof.
- Solvents present in even small amounts can cause irritation of the oral mucosa in sensitive individuals. Utilising only water reduces this risk.
- No environmental issues with solvent emissions.

According to a eighth aspect of the present invention there is provided use of a film as defined in any of the first, second, third, fourth and fifth aspects for providing tooth remineralisation.

According to a ninth aspect of the present invention there is provided a method of treating teeth by providing tooth remineralisation using a film as defined in any of the first, second, third, fourth and fifth aspects of the present invention.

### BRIEF DESCRIPTION OF THE DRAWINGS

Embodiments of the present invention will now be described, by way of example only, with reference to the accompanying drawings in which:
Figure 1 shows the enamel hardening resulting from use of the four compositions (1 to 4) according to an embodiment of the present invention;
Figures 2a and 2b are SEM images of pre-formed white spot lesions according to an embodiment of the present invention;
Figures 3a and 3b are SEM images of enamel treated with negative control film (composition 2) according to an embodiment of the present invention;
Figures 4a and 4b are SEM images of enamel treated with comparison MI paste (composition 4) according to an embodiment of the present invention;
Figures 5a and 5b are SEM images of enamel treated with the NovaMin composition of the invention according to an embodiment of the present invention;
Figures 6a and 6b are SEM images of enamel treated with comparison SootheRx (composition 3) according to an embodiment of the present invention.
Figure 7 shows an SEM image of an etched dentine surface;
Figure 8 is an SEM image of an etched dentine surface treated with a polymer film containing NovaMin according to an embodiment of the present invention;
Figure 9 shows the comparison between a treated and untreated dentin surface; and
Figure 10 details results from an *in-vitro* study on the effects of NovaMin containing films on the growth of oral bacteria which are associated with caries development and periodontal disease.

### BRIEF DESCRIPTION

Generally speaking, the present invention resides in the provision of a remineralisation composition in the form of a film, which may be adhered to one or more teeth, and provides a convenient and unobtrusive method of generating a high and sustained local concentration of remineralising components on the tooth surface. When hydrated, the film releases remineralising ions forming a stable crystalline hydroxycarbonate apatite layer on the tooth surface, providing long term reduction of tooth sensitivity. The film also has the ability to inhibit the growth of oral bacteria associated with caries development and periodontal disease.

The film comprises a water soluble film former such as a polymeric film former and a bioactive glass.

### EXAMPLES

### Surface Microhardness Testing

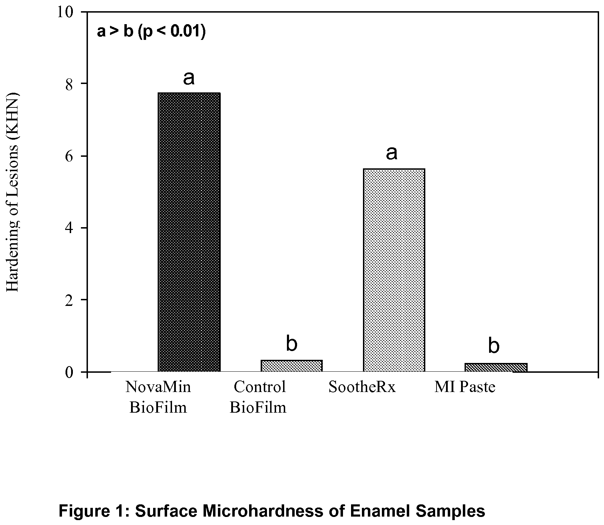

The results of surface microhardness testing are shown in Figure 1. The results are presented as relative hardening of white spot lesions (i.e. all data have been normalised to the measured hardness of lesionised enamel). Treatment with a 16% by weight NovaMin film according to the invention and a 7.5% by weight NovaMin toothpaste (SootheRx, positive control) resulted in a significant hardening of the artificial white spot lesions. Treatment with the control BioFilm samples (negative control) and MI Paste (competitive product, contains Recaldent) did not change the surface hardness of the lesions.

### SEM Analysis

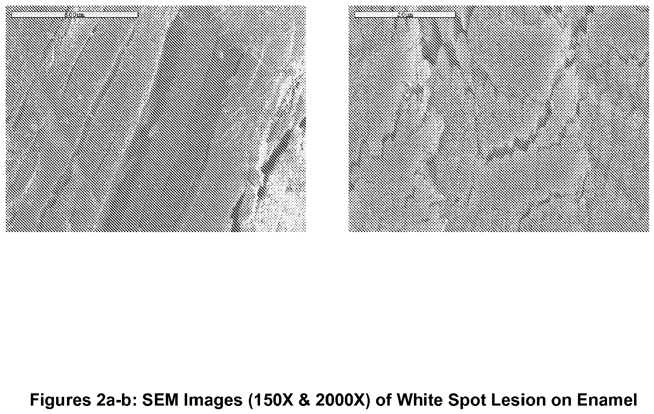

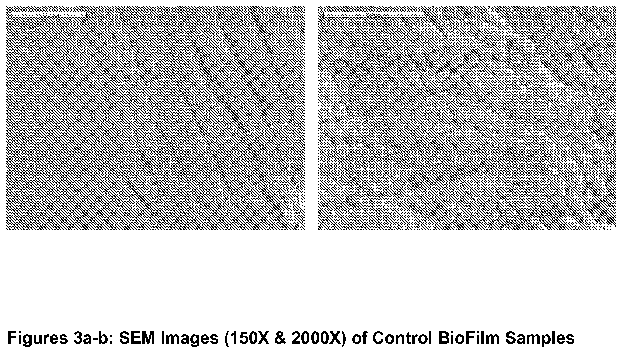

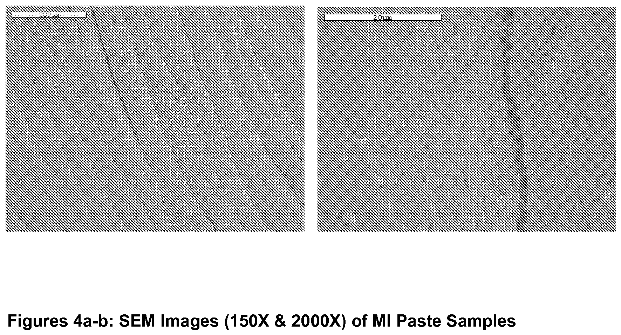

Figures 2a-b are scanning electromicrograph (SEM) images of pre-formed white spot lesions on bovine enamel. Figure 2a is a low-magnification view of the severe surface disruption that was caused by the lesion formation. Figure 2b is a high-magnification image of the prismatic structure of enamel that remained intact along the edges of the white spots and within the "bars" of enamel seen in Figure 2a. Images of surfaces treated with control BioFilm samples (Figures 3a - b) and those treated with MI Paste (Figures 4a - b) show the same features.

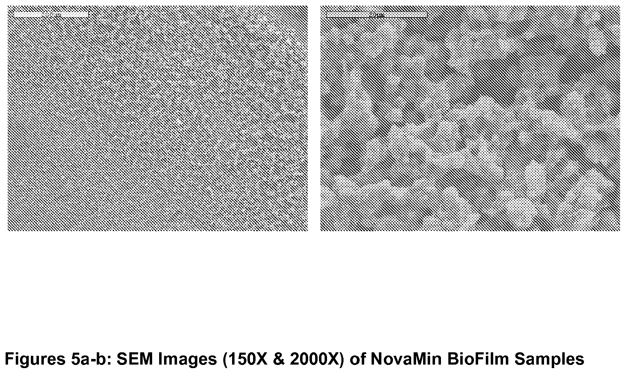

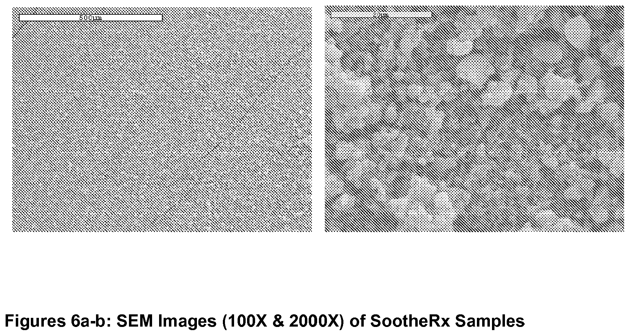

Figures 5a-b are images of the lesionised enamel treated with the 16% NovaMin BioFilm samples according to the invention. At low magnification (Figure 5a) it is clear that a large amount of NovaMin particulate has been deposited on the surface of the lesions (especially along the edges). A higher magnification view (Figure 5b) shows that the NovaMin particulate has adhered to the tissue and begun to form a tenacious hydroxy carbonate apatite layer that is chemically similar to native tooth mineral and which completely obscures the view of the lesionised enamel surface. Images of samples treated with SootheRx (Figures 6a - b) show that treatment with a 7.5% NovaMin toothpaste yielded very similar results.

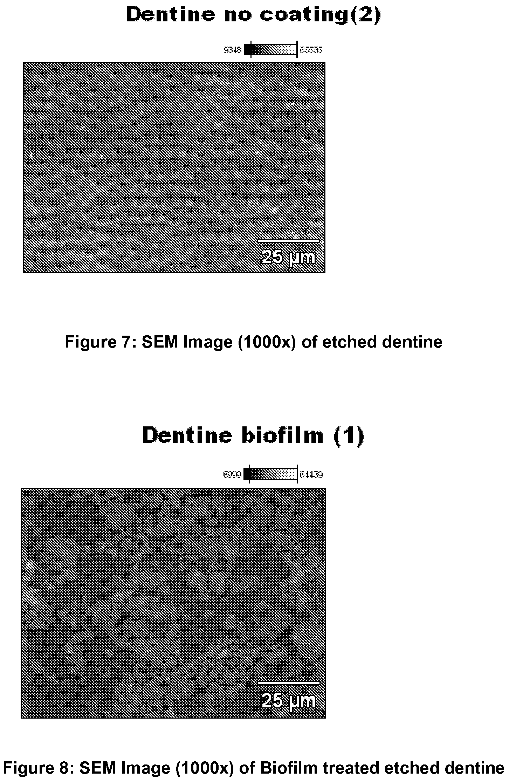

Figures 7 shows untreated dentine with open tubules clearly visible. Figure 8 shows a similarly prepared dentine surface that has been treated with a single application of a polymer film containing NovaMin particulate. It is evident that the polymer film has deposited bioactive glass on the dentine surface.

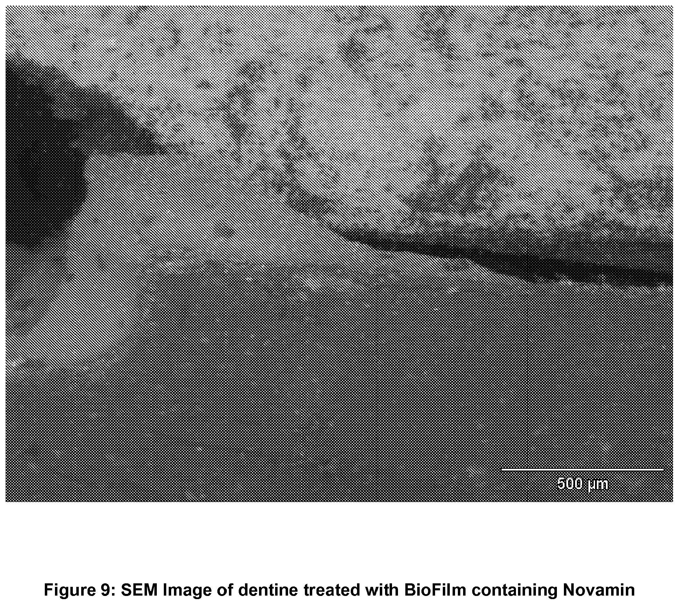

Figure 9 shows a dentine slab which has been split in two, the upper half treated with a polymer film containing NovaMin, and the lower portion remaining untreated. Areas highlighted in green, yellow and orange highlight where Novamin has been deposited.

### Inhibition of Bacterial Growth

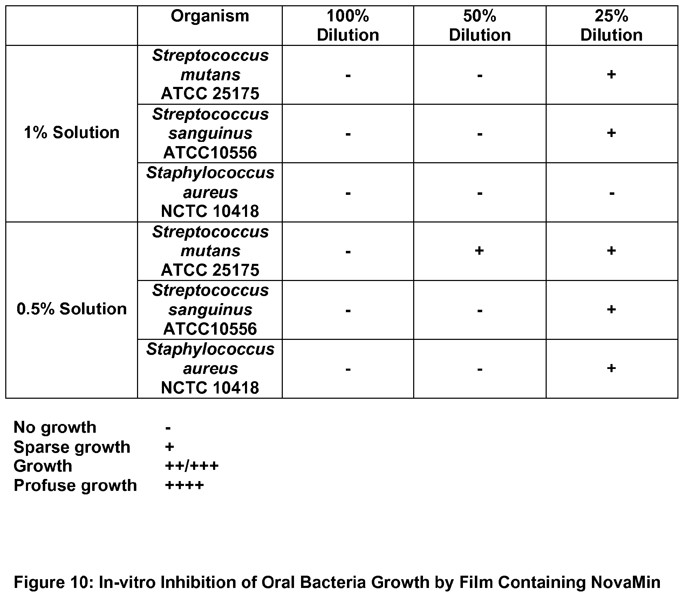

The results from *in-vitro* testing of films containing NovaMin particulate and their ability to inhibit the growth of oral bacteria are detailed in Figure 10. Aqueous solutions of 1.0% and 0.5% NovaMin were prepared from resorbable hydroxypropyl methylcellulose polymer strips that contained 15% NovaMin. Six sets of 100%, 50%, and 25% serial dilutions were prepared from each solution, and 5ml aliquots inoculated with approximately 0.02ml of *Streptococcus mutans* ATCC 25175, *Streptococcus sanguinus* ATCC10556, and *Staphylococcus aureus* NCTC 10418. Positive and negative controls were also prepared. All test dilutions and controls were incubated at overnight at 35 °C and then streaked onto tryptone soy agar (TSA) plates. The agar plates were then incubated at 35 °C overnight and examined for growth. At full strength (100%) the 1.0% and 0.5% solutions completely inhibited growth of all three bacterial species. Dilution of the solutions slightly diminished the inhibitory effect, but this was only sparse compared to the control.

### CONCLUSION

The results presented here indicate that a resorbable polymer strip containing 16% NovaMin particulate according to the invention (Test Product 1) has potential to effectively treat white spot lesions that have formed on enamel surfaces. Treatment with the NovaMin-containing BioFilm samples had a similar effect as treatment with NovaMin-containing toothpaste (Test Product 3 - comparison) and, in this model, greatly outperformed polymer-only resorbable strips (Test Product 2) and Recaldent-containing toothpaste (Test Product 4).

However, in practical use it is to be expected that the film according to the present invention would remain in place on the tooth for a longer period of time than the toothpaste (comparison); leading to greater remineralisation efficacy.

It has also been demonstrated that a resorbable polymer product containing NovaMin has the potential to inhibit the growth of cariogenic and periodontal bacteria on the tooth surfaces and adjacent gingival tissues.

Whilst specific embodiments of the present invention have been described above, it will be appreciated that departures from the described embodiments may still fall within the scope of the present invention. For example, any suitable type of soluble film former and bioactive glass may be used.

## Claims

1. A film for use in an oral cavity wherein said film is produced from an aqueous mixture of its components and comprises:
a water soluble polymeric film former which is an edible non-ionic cellulose derivative selected from any one or combination of methyl celluloses, hydroxypropylmethyl cellulose, hydroxyethylcellulose, hydroxypropyl cellulose and carboxymethyl cellulose;
and a bioactive glass comprising 40-80% by weight silicon dioxide (SiO₂), 0-35% by weight sodium oxide (Na₂O), 4-46% by weight calcium oxide (CaO) and about 1-15% by weight phosphorous oxide (P₂O₅);
and wherein the bioactive glass is added to a solution of the polymeric film former to form the aqueous mixure and the bioactive glass is held in this aqueous environment for no longer than 1-120 hours or more preferably 1-60 hours before the water content is reduced by 5-70% by weight so as to minimize adverse reactions between the polymeric film former and the bioactive glass.

2. A film for use in an oral cavity according to claim 1 wherein said film is produced from an aqueous mixture of its components and wherein the mixture of the water soluble polymeric film former and the bioactive glass is passed through a high shear mixer to produce a homogenous dispersion which in turn is deaerated before being coated onto a carrier paper and air dried at above room temperatures.

3. A film for use in an oral cavity according to claim 1 or claim 2 wherein the edible non-ionic cellulose derivative is selected from hydroxypropyl cellulose and hydroxypropylmethyl cellulose.

4. A film for use in an oral cavity according to claim 1 or claim 2, wherein the film comprises water soluble polymeric film former in an amount of 1-90%, 20-80%, 30-70%, 40-80% or 50-60% by weight of the film.

5. A film for use in an oral cavity according to claim 1 or claim 2 , wherein particles forming the bioactive glass have an average particle size of less than 100 microns, less than 75 microns, less than 50 microns, less than 20 microns, less than 10 microns, or less than 5 microns

6. A film according to any one of claims 1 to 5 for remineralizing teeth.

## Patentansprüche

1. Schicht zur Verwendung in einem Hohlraum der Mundhöhle, wobei die Schicht aus einer wässrigen Mischung ihrer Bestandteile hergestellt wird und umfasst:
einen wasserlöslichen Polymerschichtbildner, der ein essbares, nicht-ionisches Cellulose-Derivat ist, das aus einem oder einer Kombination aus Methyl-Cellulosen, Hydroxypropylmethyl-Cellulose, Hydroxyethyl-Cellulose, Hydroxypropyl-Cellulose und Carboxymethyl-Cellulose ausgewählt ist;
und ein bioaktives Glas, das 40-80 Gew.-% Siliciumdioxid (SiO₂), 0-35 Gew.-% Natriumoxid (Na₂O), 4-46 Gew.-% Calciumoxid (CaO) und etwa 1-15 Gew.-% Phosphoroxid (P₂O₅) umfasst;
und wobei das bioaktive Glas zu einer Lösung des Polymerschichtbildners hinzugegeben wird, um die wässrige Mischung zu bilden, und das bioaktive Glas in dieser wässrigen Umgebung für nicht länger als 1-120 Stunden oder bevorzugter 1-60 Stunden gehalten wird, bevor der Wassergehalt um 5-70 Gew.-% reduziert wird, um nachteilige Reaktionen zwischen dem Polymerschichtbildner und dem bioaktiven Glass zu minimieren.

2. Schicht zur Verwendung in einem Hohlraum der Mundhöhle gemäß Anspruch 1, wobei die Schicht aus einer wässrigen Mischung ihrer Bestandteile hergestellt wird und wobei die Mischung des wasserlöslichen Polymerschichtbildners und des bioaktiven Glases durch einen Mischer mit hoher Scherung geleitet wird, um eine homogene Dispersion herzustellen, die wiederum entgast wird, bevor sie auf ein Trägerpapier beschichtet wird und oberhalb der Raumtemperatur luftgetrocknet wird.

3. Schicht zur Verwendung in einem Hohlraum der Mundhöhle gemäß Anspruch 1 oder Anspruch 2, wobei das essbare, nicht-ionische Cellulose-Derivat aus Hydroxypropyl-Cellulose und Hydroxypropylmethyl-Cellulose ausgewählt ist.

4. Schicht zur Verwendung in einem Hohlraum der Mundhöhle gemäß Anspruch 1 oder Anspruch 2, wobei die Schicht wasserlöslichen Polymerschichtbildner in einer Menge von 1-90, 20-80, 30-70, 40-80 oder 50-60 Gew.-% bezogen auf die Schicht umfasst.

5. Schicht zur Verwendung in einem Hohlraum der Mundhöhle gemäß Anspruch 1 oder Anspruch 2, wobei Partikel, die das bioaktive Glas bilden, eine durchschnittliche Partikelgröße von weniger als 100 Mikrometer, weniger als 75 Mikrometer, weniger als 50 Mikrometer, weniger als 20 Mikrometer, weniger als 10 Mikrometer oder weniger als 5 Mikrometer aufweisen.

6. Schicht gemäß einem der Ansprüche 1 bis 5 zur Remineralisierung der Zähne.

## Revendications

1. Film pour son utilisation dans une cavité buccale, dans lequel ledit film est produit à partir d'un mélange aqueux de ses composants et comprend :
un agent filmogène polymère soluble dans l'eau qui est un dérivé de cellulose non ionique comestible choisi parmi l'une quelconque ou une combinaison de méthylcelluloses, d'hydroxypropylméthylcellulose, d'hydroxyéthylcellulose, d'hydroxypropylcellulose et de carboxyméthylcellulose ;
et un verre bioactif comprenant 40 à 80 % en poids de dioxyde de silicium (SiO₂), 0 à 35 % en poids d'oxyde de sodium (Na₂O), 4 à 46 % en poids d'oxyde de calcium (CaO) et environ 1 à 15 % en poids d'oxyde phosphoreux (P₂O₅) ;
et dans lequel le verre bioactif est ajouté à une solution de l'agent filmogène polymère pour former le mélange aqueux et le verre bioactif est maintenu dans cet environnement aqueux pendant pas plus longtemps que 1 à 120 heures et de manière davantage préférée de 1 à 60 heures avant que la teneur en eau soit réduite de 5 à 70 % en poids de façon à réduire au maximum les réactions indésirables entre l'agent filmogène polymère et le verre bioactif.

2. Film pour son utilisation dans une cavité buccale selon la revendication 1, dans lequel ledit film est produit à partir d'un mélange aqueux de ses composants et dans lequel le mélange de l'agent filmogène polymère soluble dans l'eau et du verre bioactif est passé à travers un mélangeur à cisaillement élevé pour produire une dispersion homogène qui est elle-même désaérée avant d'être appliquée sur un papier de support et séchée à l'air à des températures supérieures à la température ambiante.

3. Film pour son utilisation dans une cavité buccale selon la revendication 1 ou la revendication 2, dans lequel le dérivé de cellulose non ionique comestible est choisi parmi l'hydroxypropylcellulose et l'hydroxypropylméthylcellulose.

4. Film pour son utilisation dans une cavité buccale selon la revendication 1 ou la revendication 2, dans lequel le film comprend un agent filmogène polymère soluble dans l'eau en une quantité de 1 à 90 %, 20 à 80 %, 30 à 70 %, 40 à 80 % ou 50 à 60 % en poids du film.

5. Film pour son utilisation dans une cavité buccale selon la revendication 1 ou la revendication 2, dans lequel des particules formant le verre bioactif ont une taille moyenne de particule inférieure à 100 microns, inférieure à 75 microns, inférieure à 50 microns, inférieure à 20 microns, inférieure à 10 microns ou inférieure à 5 microns.

6. Film selon l'une quelconque des revendications 1 à 5, pour la reminéralisation des dents.
